# FASCICULE DE BREVET EUROPEEN

(11) **EP 2 833 814 B1**
(45) Date de publication et mention de la délivrance du brevet: **08.06.2022**
(21) Numéro de dépôt: 13719965.9
(22) Date de dépôt: 02.04.2013
(51) Int. Cl.: A61B 17/88, B21D 7/06

(54) **CINTREUSE À CAME POUR TIGE ORTHOPÉDIQUE**
BIEGEMASCHINE MIT EINEM NOCKEN FÜR EINE ORTHOPÄDISCHE STANGE
BENDING MACHINE WITH A CAM FOR AN ORTHOPAEDIC ROD

(30) Priorité: 02.04.2012 FR 1252995
(43) Date de publication de la demande: 11.02.2015
(73) Titulaire: Safe Orthopaedics, 95610 Eragny-sur-Oise (FR)
(72) Inventeur: PETIT, Dominique, 95610 Eragny sur Oise (FR); LE HUEC, Jean Charles, 33600 Pessac (FR)
(74) Mandataire: IP Trust
(86) Numéro de dépôt international: PCT/FR2013/050721
(87) Numéro de publication internationale: WO 2013/150233

(56) Documents cités:
- FR-A- 1 342 386
- US-A- 3 824 834
- US-A- 4 389 872
- US-A- 4 474 046
- US-A- 5 490 409
- US-A1- 2009 254 326

## Description

### Domaine de l'invention

La présente invention concerne le domaine des outils de cintrage manuel de tiges, notamment pour le cintrage de tiges orthopédiques telles que des éléments de liaison intervertébraux.

Ces tiges sont typiquement des tiges en acier ou en titane, d'une section de cinq à six millimètres. Les rayons de cintrage recherchés sont habituellement compris entre vingt à cent millimètres. Le cintrage nécessite l'exercice d'un effort très important de plusieurs milliers de Newton, notamment pour les tiges en titane.

### Etat de la technique

On connaît dans l'état de la technique plusieurs brevets décrivant des outils de cintrage manuels constitués de deux bras articulés supportant un galet principal et deux galets secondaires montés chacun à l'extrémité de l'un des deux bras. De tels outils sont décrits dans les brevets américains US3824834, US4474046, US5490409 ou US5819580.

Ces brevets proposent l'utilisation d'un galet principal ajustable pour optimiser l'angle de cintrage de la tige.

### Inconvénients de l'art antérieur

Ces outils de cintrage manuel présentent plusieurs inconvénients.

En premier lieu, la mise en œuvre des outils de cintrage connu constitue un exercice physique important, peu compatible avec une manipulation précautionneuse et précise : l'opérateur doit exercer sur les branches un effort à la limite de ses forces, ce qui l'oblige à concentrer son attention à l'exercice de ces efforts, plutôt qu'au positionnement précis de la tige à cintrer et à la vérification permanente du maintien d'un positionnement correct. Lorsque l'effort est relâché, il arrive que la tige sorte de la tête de l'outil et choit, ce qui est particulièrement problématique lorsqu'il s'agit de tiges orthopédiques nécessitant impérativement la préservation de la stérilité.

Un deuxième problème est celui des limites de l'angle de cintrage. Les outils de l'art antérieur ne permettent pas des cintrages avec des rayons de courbure très faibles, et sont généralement limités à des rayons de courbure supérieure à une cinquantaine de millimètres.

Un troisième problème est celui du poids et le nettoyage de ces outils. Afin de résister aux efforts importants appliqués par l'utilisateur, il s'agit généralement d'outils présentant des bras métalliques massifs, et des articulations présentant des zones d'accumulation de salissures.

### Solution apportée par l'invention

Afin de remédier à ces problèmes, l'invention concerne selon son acception la plus générale une cintreuse à came constituée par deux bras articulés, comportant un galet principal coaxial avec l'axe d'articulation desdits bras, l'extrémité de chacun desdits bras présentant des moyens pour exercer un effort de cintrage sur une tige caractérisé en ce que lesdits moyens sont constitués par des œillets fermés, chacun desdits œillets étant monté à l'extrémité de la partie courte de l'un desdits bras de manière mobiles en rotation par rapport à un axe parallèle à l'axe dudit galet principal.

On entend par « partie courte » du bras, la partie s'étendant en avant du point d'articulation. Le point d'articulation se trouve entre une partie courte supportant les œillets et une partie longue servant à exercer une force, et formant levier amplificateur d'effort.

Avantageusement, chaque œillet comporte une lumière d'axe perpendiculaire à l'axe de rotation de l'œillet, la lumière étant agencée pour permettre le passage de la tige à cintrer.

Selon un mode de réalisation particulier, chaque œillet est constitué par une pièce cylindrique pourvue en extrémité d'une tête traversée par une lumière, ladite pièce étant montée libre en rotation au travers d'un alésage ménagé à l'extrémité de la partie courte (5, 6) desdits bras. Ainsi configurés, les œillets assurent un guidage axial de la tige au cours de l'opération de cintrage permettant un cintrage de la tige important et uniforme.

De préférence, les deux bras sont superposés.

Avantageusement, l'œillet monté sur le bras inférieur présente une embase d'une épaisseur correspondant à l'épaisseur du bras supérieur, afin que les centres des lumières des deux œillets soient coplanaires.

Selon une autre variante avantageuse, les deux bras sont plats, et sont orientés parallèlement au plan médian.

De préférence, l'angle formé entre la droite passant entre l'axe de rotation de l'un des œillets et l'axe de rotation du galet principal d'une part, et l'axe passant entre l'axe de rotation d'un galet principal et la zone de préhension du bras correspondant d'autre part, est compris entre 75° et 100°, de préférence d'environ 90°

Avantageusement, la partie du bras opposée à l'œillet forme un angle divergent, c'est-à-dire que le segment arrière du bras n'est pas dans le prolongement du segment principal du manche, mais dévie vers l'extérieur.

Selon une variante, le galet principal présente une gorge d'un rayon sensiblement égal à celui de la tige à cintrer, dont le centre est coplanaire avec le centre des lumières formées dans les œillets.

Selon un mode de réalisation préféré, les bras sont configurés pour permettre le croisement de leurs zones intermédiaires.

Selon un mode de réalisation particulier, les bras sont réalisés en un polymère, de préférence stérile, pour la réalisation d'un outil à usage unique.

Selon une variante de réalisation, chaque tête supporte à son extrémité traversée par l'axe de rotation de l'œillet un prolongement vertical, coaxial avec ledit axe de rotation, d'une hauteur correspondant à l'épaisseur de la tête du bras complémentaire, ladite tête comprenant en outre un prolongement radial, s'étendant parallèlement à la zone triangulaire, entre l'axe de rotation de l'œillet et l'axe de rotation du galet principal pour former une fourchette permettant de recevoir la zone triangulaire de la tête complémentaire pour former une charnière.

Avantageusement, le prolongement vertical, le prolongement radial et la tête sont réalisés de manière monolithique par moulage d'une pièce unique.

Selon une alternative, ladite tête présente une forme triangulaire s'étendant vers l'extérieur.

Selon une autre variante, l'un au moins des manches présente des marquages représentatifs du rayon de courbure atteint.

### Description d'un mode de réalisation détaillé non limitatif

D'autres objets et avantages de l'invention apparaîtront au cours de la description qui suit, faite en référence aux dessins annexés, concernant un exemple non limitatif de réalisation d'une cintreuse selon l'invention, dans lesquels :
- la figure 1 représente une vue d'une cintreuse en position neutre
- la figure 2 représente une vue de face de la tête de la cintreuse selon l'invention
- la figure 3 représente une vue de ladite cintreuse, avec une tige en début de cintrage
- la figure 4 représente une vue de ladite cintreuse, avec une tige en fin de cintrage
- la figure 5 représente une vue de ladite cintreuse, avec une tige ayant subi une succession de cintrages
- la figure 6 représente une vue de trois quart avant d'une variante de réalisation d'une cintreuse selon l'invention
- la figure 7 représente une vue en perspective de la tête d'un bras selon cette variante.

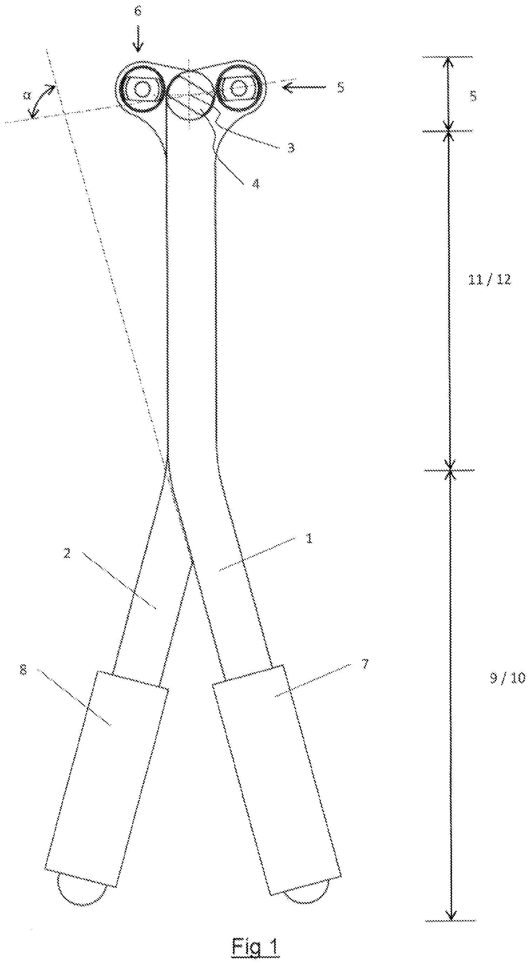

La figure 1 représente un exemple non limitatif de réalisation d'une cintreuse selon l'invention. Elle est constituée par un bras supérieur (1) et un bras inférieur (2) articulés par un axe de rotation (3) supportant également un galet principal (4).

Chacun des bras (1, 2) est constitué par une tête (5, 6) de cintrage et un manche (7, 8). Les bras (1, 2) sont découpés dans une plaque métallique d'une épaisseur de 3 millimètres. Ils peuvent également être réalisés par moulage d'une matière plastique chargé de fibres de carbone ou de fibres de verre. Les surfaces médianes de ces bras (1, 2) sont parallèles et planes de façon à permettre le glissement d'un bras sur l'autre, sur l'ensemble du débattement autorisé.

Les bras présentent une largeur sensiblement constante, de seize millimètres par exemple, pour des bras métalliques, en acier.

Le manche (7, 8) des bras (1, 2) est gainé dans sa partie arrière pour faciliter la préhension. L'épaisseur du gainage est déterminée pour ne pas gêner la superposition des bras. Le cas échéant, le gainage ne s'étend pas sur les surfaces en regard des bras (1, 2).

Le manche (7, 8) présente une forme divergente, avec une partie arrière (9, 10) formant un angle d'environ 160° avec la partie avant (11, 12).

La longueur de la partie arrière (9, 10) est d'environ 150 millimètres, et la longueur de la partie avant (11, 12) est d'environ 100 millimètres. La longueur de la partie arrière (9, 10) correspond sensiblement à 1,5 fois longueur de la partie avant (11, 12).

En début de cintrage, correspondant à la phase où les efforts à exercer son maximum, l'ouverture de la partie arrière (9, 10) est d'environ 100 millimètres, pour une bonne préhension de l'instrument. Il est ainsi possible d'exercer une action avec une seule main.

Les parties courtes (5, 6) des bras (1, 2) présentent une longueur d'environ 25 millimètres. Elles supportent chacune un œillet (13, 14) rotatif par rapport à un axe parallèle à l'axe de rotation (3) du galet principal (4).

Dans le mode de réalisation illustré, chaque œillet (13, 14) est constitué par une pièce cylindrique prolongée à l'une de ses extrémités par une tête (130, 140) plate de section inférieure à la section de ladite pièce. La tête (130, 140) est traversée par une lumière diamétrale (15, 16) d'une section légèrement supérieure à celle de la tige à cintrer. A titre d'exemple, la section de la lumière diamétrale (15, 16) est de 6 millimètres pour des tiges à cintrer d'une section de 5,5 millimètres. Le jeu permet non seulement d'introduire facilement la tige, mais aussi de la déplacer et de la retirer nonobstant les déformations appliquées lors du cintrage. La pièce cylindrique est montée au travers d'un alésage ménagé au niveau de l'extrémité de la partie courte (5, 6) du bras associé (1, 2) et présentant un axe parallèle à l'axe du galet principal (4). Elle présente une section légèrement inférieure à celle de l'alésage de manière à laisser un jeu permettant la rotation de l'œillet dans l'alésage.

Avantageusement, la lumière (15, 16) de chaque œillet (13, 14) est agencée pour présenter un axe perpendiculaire à l'axe de rotation de l'oeillet. Du fait que les oeillets (13, 14) sont montés libres en rotation sur les bras, l'axe des lumières (15, 16) reste sensiblement perpendiculaire à l'axe de la tige (19) durant l'opération de centrage. Ainsi, durant l'opération de cintrage, la tige, sous l'action de l'effort de cintrage appliqué par le galet principal (4), va coulisser axialement à l'intérieur des lumières.

Selon un mode de réalisation particulier, chaque œillet (13, 14) est conformé et agencé par rapport au galet principal (4) pour présenter, lorsque la tête (130, 140) de chaque oeillet est positionnée pour présenter la lumière (15, 16) associée en regard du galet principal (4), un écartement permettant le passage de la tige (19) entre le galet principal (4) et les oeillets (13, 14). Le pourtour extérieur de chaque tête (130, 140) s'étendant dans un plan perpendiculaire à l'axe des lumières (12, 14) constitue alors une zone d'appui permettant, avec le galet principal (4), le cintrage de la tige. Avantageusement, la surface d'appui de chaque tête (130, 140) est pourvue d'une gorge de rayon sensiblement égal à celui de la tige à cintrer. Selon une configuration avantageuse, le pourtour extérieur de chaque œillet présente au moins une zone d'appui plane, la zone restante étant circulaire. Pourvu ainsi de deux zones distinctes, l'œillet offre deux rayons de cintrage distincts de la tige. Il est bien entendu évident que le nombre de zones d'appui ne se limite pas à un ou deux, et qu'il peut être prévu un pourtour offrant plus de deux zones distinctes selon le nombre de rayons de cintrage souhaité.

L'angle formé entre la droite passant entre l'axe de rotation de l'un des œillets et l'axe de rotation du galet principal d'une part, et l'axe passant entre l'axe de rotation d'un galet principal et la zone de préhension du bras correspondant d'autre part, est d'environ 90°.

Le galet principal (3) ainsi que les œillets (13, 14) sont fixés par des écrous. Ces écrous sont éventuellement démontables pour permettre le remplacement des œillets, notamment pour adapter à la taille des tiges à cintrer.

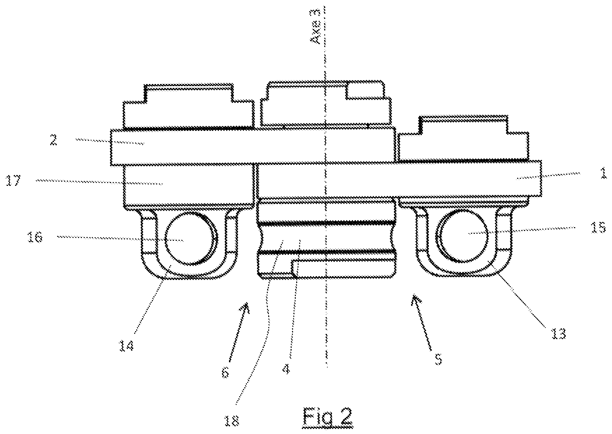

Comme représenté en figure 2, afin de rattraper le décalage résultant de l'épaisseur des bras, l'œillet (14) monté sur le bras inférieur (2) présente une embase (17) d'une épaisseur sensiblement égale à celle du bras (2).

Le galet principal (3) est optionnellement libre en rotation. Il présente une gorge (18) dont le rayon de courbure est de 2,8 millimètres, sensiblement identique voire légèrement supérieur à celui de la tige à cintrer.

La distance entre l'axe (3) du galet principal d'une part, et l'axe de chacun des œillets d'autre part, et minimal et limité par la section de l'œillet et du galet principal, et le cas échéant de l'embase de l'œillet (17) et éventuellement des écrous. Dans l'exemple décrit, cette distance est de 18 millimètres.

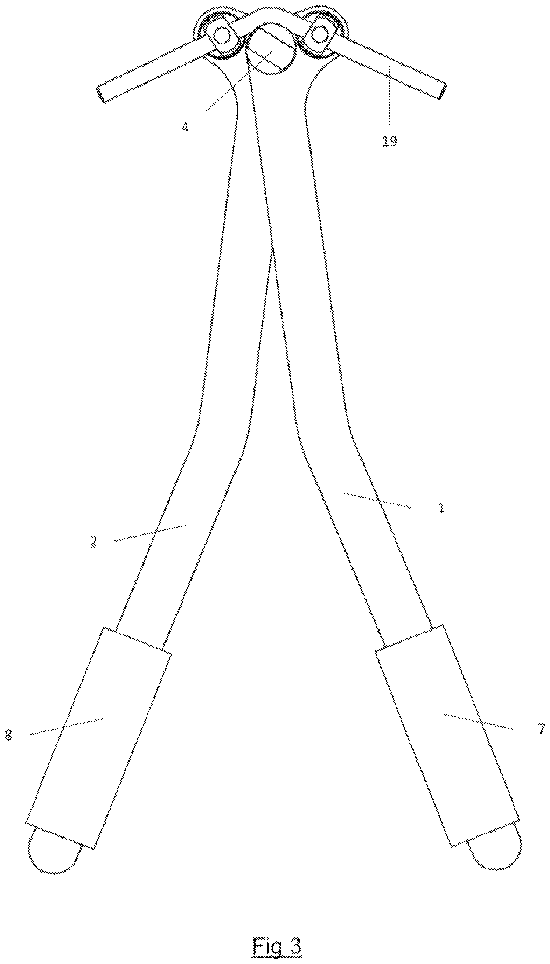

La figure 3 représente une vue de la cintreuse en début de cintrage. la tige de liaison intervertébrale (19) est glissée dans les deux lumières des œillets et glisse dans la gorge du galet principal (4). Les deux bras sont écartés suffisamment pour libérer un chemin de passage de la tige (19).

A partir de ce moment, la tige de liaison (19) est maintenue en place, et ne peut tomber même en cas de manipulation maladroite de la cintreuse (sauf dans la situation très improbable d'une orientation verticale de l'outil dans une configuration où les deux lumières sont alignés verticalement et où aucun effort n'est exercé sur les bras).

En rapprochant les manches (7, 8) des deux bras (1, 2), on exerce une contrainte par l'intermédiaire des deux œillets et du galet principal, avec un effet de levier important, provoquant la déformation de la tige à cintrer (19). Dans l'exemple décrit, l'amplification des efforts est environ de 12 fois.

Le niveau d'amplification est déterminé par le rapport entre la longueur partie arrière (9, 10) calculée entre le point d'articulation supportant l'axe de rotation (3) du galet principal, et la longueur de la parties courtes (5, 6) calculée entre ce point d'articulation supportant l'axe de rotation (3) du galet principal (4) et l'axe de rotation de l'œillet (13, 14) supporté par le bras.

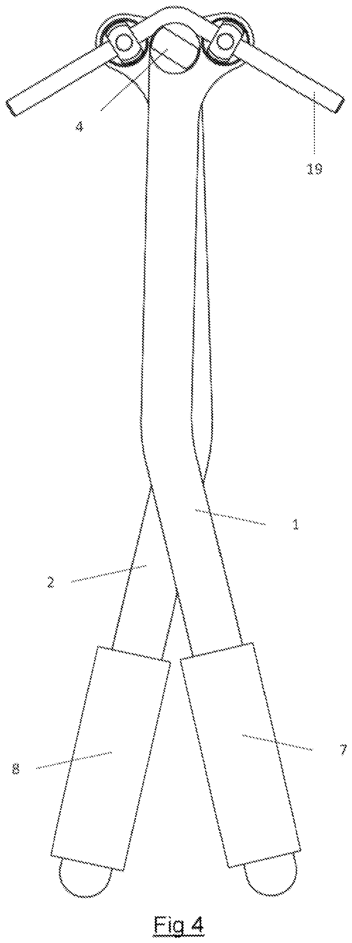

La figure 4 représente la situation où on cherche un rayon de courbure particulièrement faible. La configuration des manches (7, 8) permet un débattement important, dépassant la position dans laquelle les bras sont parallèles. Les bras peuvent se croiser, ce qui permet d'augmenter l'amplitude du débattement.

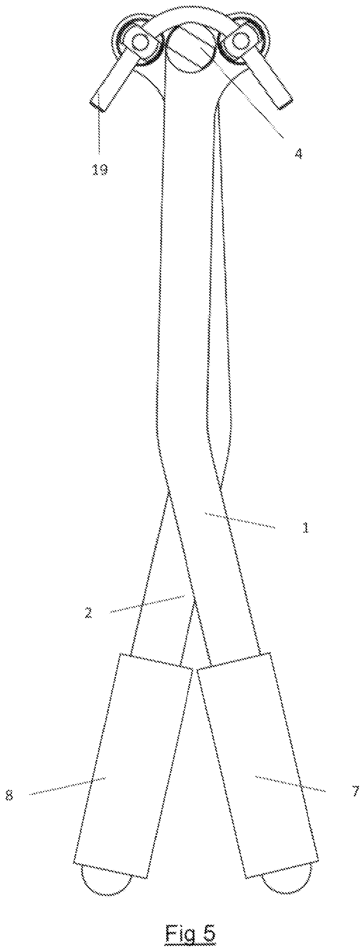

La figure 5 représente la situation où la tige (19) fait l'objet d'une succession de centrages par déplacements régulier de la tige. On exerce un premier cintrage, puis on déplace longitudinalement la tige par rapport aux œillets et au galet de quelques millimètres avant d'exercer une nouvelle étape de cintrage, et ainsi de suite.

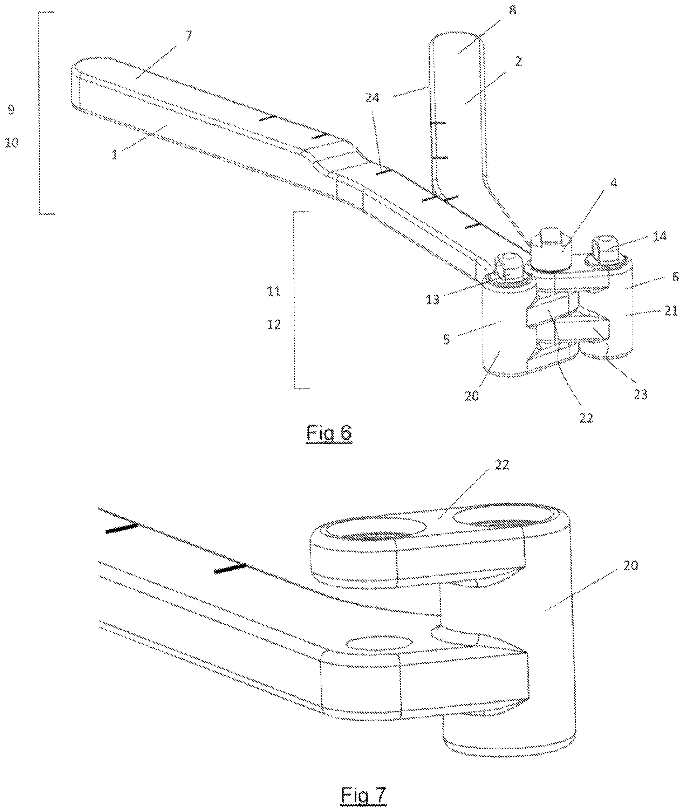

Les figures 6 et 7 concernent une variante de l'invention, représentant respectivement une vue de trois-quart avant d'une cintreuse selon l'invention et une vue en perspective de la tête d'un bras selon cette variante.

Dans cette réalisation, les bras (1, 2) ne sont pas constitués par des éléments plats métalliques, mais par des éléments moulés à section sensiblement rectangulaire. La section rectangulaire est déterminée pour présenter une largeur (mesurée dans un plan parallèle au plan médian de l'instrument) supérieure à l'épaisseur (mesurée selon un axe parallèle à l'axe de rotation du galet principal). Les bras peuvent être réalisés en un polymère chargé pour des applications d'instruments chirurgicaux à usage unique.

Comme dans la variante précédente, les deux bras (1, 2) sont identiques. Cette caractéristique n'est toutefois pas nécessaire, il serait possible de réaliser un instrument avec deux bras différents et complémentaires. Cette caractéristique est toutefois préférée car elle permet de réduire le coût de fabrication, par l'utilisation d'un seul moule commun pour le bras droit et le bras gauche, et de surcroit elle aboutit à des constructions très solides et fiables du point de vue mécanique.

Chacun des bras (1, 2) présente comme dans la première variante une partie arrière (9, 10) dont le segment arrière forme les manches respectivement (7, 8). Ces manches (7, 8) sont légèrement divergent vers l'extérieur, avec un angle d'environ 15° par rapport au segment avant (11, 12), compris entre l'axe de rotation (3) et le manche. Ces manches (7, 8) présentent une épaisseur supérieure au segment avant (11, 12) afin d'améliorer le confort d'utilisation et renforcer le bras de levier soumis à des efforts importants lors du cintrage. L'épaisseur du manche (7, 8) est d'environ 15 millimètres, alors que l'épaisseur du segment avant (11, 12) est de 10 millimètres. Le manche (7, 8) peut présenter une forme ergonomique adaptée à la main d'un utilisateur, par exemple une forme de poignée avec des évidements cannelés.

Chaque tête (5, 6) présente dans l'exemple décrit une forme triangulaire s'étendant vers l'extérieur. Cette zone triangulaire supporte à son extrémité traversée par l'axe de rotation de l'œillet respectivement (13, 14) un prolongement vertical (20, 21), coaxial avec ledit axe de rotation, d'une hauteur correspondant à l'épaisseur de la tête (6, 5) du bras complémentaire.

La tête (5, 6) comprend en outre un prolongement radial (22, 23), s'étendant parallèlement à la zone triangulaire, entre l'axe de rotation de l'œillet (13, 14) et l'axe de rotation (3) du galet principal.

Le prolongement radial (22, 23) et la zone triangulaire de la tête (5, 6) forme une fourchette permettant de recevoir la zone triangulaire de la tête complémentaire (6, 5) pour former une charnière.

Le prolongement vertical (20), le prolongement radial (22) et la zone triangulaire peuvent être réalisés de manière monolithique par moulage d'une pièce unique formant un bras moulé en polymère chargé.

Il est aussi possible de réaliser le bras par assemblage de trois pièces distincts, à savoir un prolongement radial constitué par une bague d'une épaisseur correspondant à celle de la zone triangulaire, et un prolongement radial constitué par une pièce rectangulaire présentant deux évidements pour le passage des axes de l'œillet d'une part et du galet principal d'autre part. Dans ce cas, les axes assurent également l'assemblage des trois pièces constitutives de la tête.

Optionnellement l'un au moins des manches (7, 8) de l'une quelconque des variantes (et de préférence le bras inférieur) présente des marquages (24) permettant d'informer l'utilisateur de l'angle de courbure atteinte. La lecture se fait au niveau de l'intersection des deux bras. Le repère visible à l'intersection donne une indication sur le niveau de courbure atteint. La valeur de chacune des graduations est déterminée par un calcul géométrique prenant en compte les dimensions du bras, des galets et œillets. Elle peut également être déterminée de manière empirique, en procédant successivement à des cintrages de tige dont on mesure le rayon de courbure après relâchement de l'effort, et on note le pont d'intersection au moment de l'effort maximum, pour lui attribuer la valeur du rayon de courbure observé.

## Revendications

1. Cintreuse à came constituée par deux bras (1, 2) montés articulés l'un par rapport à l'autre autour d'un axe d'articulation, chaque bras comprenant une partie courte (5, 6) s'étendant en avant de l'axe d'articulation, chaque partie courte (5,6) présentant en extrémité des moyens pour exercer un effort de cintrage sur une tige (19), la cintreuse comportant en outre un galet principal(4) coaxial avec l'axe d'articulation (3) desdits bras (1,2), **caractérisée en ce que** les moyens pour exercer un effort de cintrage sont constitués par deux œillets (13, 14) montés respectivement mobiles en rotation sur l'extrémité de la partie courte (5, 6) de chacun desdits bras (1, 2) autour d'un axe de rotation parallèle à l'axe dudit galet principal (4), chaque œillet (13, 14) comportant une lumière (15, 16) d'axe perpendiculaire à l'axe de rotation de l'œillet, la lumière étant agencée pour permettre le passage de la tige (19) à cintrer.

2. Cintreuse selon la revendication 1, **caractérisée en ce que** chaque œillet (13, 14) est constitué par une pièce cylindrique pourvue en extrémité d'une tête traversée par une lumière (15, 16), ladite pièce étant montée libre en rotation au travers d'un alésage ménagé à l'extrémité de la partie courte (5, 6) desdits bras (1, 2).

3. Cintreuse selon la revendication 1 ou la revendication 2, **caractérisée en ce que** les deux bras (1, 2) sont superposés.

4. Cintreuse selon la revendication 3, **caractérisée en ce que** l'œillet monté sur le bras inférieur présente une embase (17) d'une épaisseur correspondant à l'épaisseur du bras supérieur, afin que les centres des lumières des deux œillets soient coplanaires.

5. Cintreuse selon l'une au moins des revendications précédentes, **caractérisée en ce que** les deux bras (1, 2) sont plats, et sont orientés parallèlement au plan médian.

6. Cintreuse selon l'une au moins des revendications précédentes, **caractérisée en ce que** l'angle formé entre la droite passant entre l'axe de rotation de l'un des œillets (13, 14) et l'axe de rotation (4) du galet principal d'une part, et l'axe passant entre l'axe de rotation d'un galet principal et la zone de préhension du bras correspondant d'autre part, est compris entre 75° et 100°.

7. Cintreuse selon l'une au moins des revendications précédentes, **caractérisée en ce que** l'angle formé entre la droite passant entre l'axe de rotation de l'un des œillets et l'axe de rotation du galet principal d'une part, et l'axe passant entre l'axe de rotation d'un galet principal et la zone de préhension du bras correspondant d'autre part, est de 90°.

8. Cintreuse selon l'une au moins des revendications précédentes, **caractérisée en ce que** chaque bras comporte une partie opposée à la partie courte portant l'œillet présentant un segment avant et un segment arrière divergent par rapport au segment avant vers l'extérieur.

9. Cintreuse selon l'une au moins des revendications précédentes, **caractérisée** en ce le galet principal (4) présente une gorge d'un rayon sensiblement égal à celui de la tige à cintrer, dont le centre est coplanaire avec le centre des lumières formées dans les œillets (13, 14).

10. Cintreuse selon l'une au moins des revendications précédentes, **caractérisée en ce que** les bras sont configurés pour permettre le croisement de zones intermédiaires desdits bras.

11. Cintreuse selon l'une au moins des revendications précédentes, **caractérisée en ce que** chaque partie courte (5, 6) supporte à son extrémité traversée par l'axe de rotation de l'œillet (13, 14) un prolongement vertical (20, 21), coaxial avec ledit axe de rotation, d'une hauteur correspondant à l'épaisseur de la partie courte (6, 5) du bras complémentaire, ladite partie courte (5, 6) comprenant en outre un prolongement radial (22, 23), s'étendant parallèlement à la zone triangulaire, entre l'axe de rotation de l'œillet (13, 14) et l'axe de rotation (3) du galet principal pour former une fourchette permettant de recevoir la zone triangulaire de la partie courte complémentaire (6, 5) pour former une charnière.

12. Cintreuse selon la revendication précédente, **caractérisée en ce que** le prolongement vertical (20), le prolongement radial (22) et la partie courte (5) sont réalisée de manière monolithique par moulage d'une pièce unique.

13. Cintreuse selon l'une au moins des revendications précédentes, **caractérisée en ce que** ladite partie courte (5, 6) présente une forme triangulaire s'étendant vers l'extérieur.

14. Cintreuse selon l'une au moins des revendications précédentes, **caractérisée en ce que** chaque bras comporte un manche (7, 8), l'un au moins desdits manches (7, 8) présentant des marquages (24) représentatifs du rayon de courbure atteint.

15. Cintreuse selon l'une au moins des revendications précédentes, **caractérisée en ce que** les bras sont réalisés par moulage en polymère.

## Patentansprüche

1. Biegevorrichtung mit Nocke, bestehend aus zwei zueinander um eine Gelenkachse gelenkig montierten Armen (1, 2), wobei jeder Arm einen sich vor der Gelenkachse erstreckenden, kurzen Abschnitt (5, 6) umfasst, wobei jeder kurze Abschnitt (5, 6) an seinem Ende Mittel zum Ausüben einer Biegekraft auf eine Stange (19) aufweist, wobei die Biegevorrichtung ferner eine zu der Gelenkachse (3) der Arme (1, 2) koaxiale Hauptrolle (4) aufweist, **dadurch gekennzeichnet, dass** die Mittel zum Ausüben einer Biegekraft aus zwei Ösen (13, 14) bestehen, die jeweils am Ende des kurzen Abschnitts (5, 6) eines jeden der Arme (1, 2) um eine zur Achse der Hauptrolle (4) parallelen Drehachse drehbar montiert sind, wobei jede Öse (13, 14) eine Öffnung (15, 16) mit einer zur Drehachse der Öse senkrechten Achse aufweist, wobei die Öffnung so angeordnet ist, dass sie ein Durchführen der zu biegenden Stange (19) zulässt.

2. Biegevorrichtung nach Anspruch 1, **dadurch gekennzeichnet, dass** jede Öse (13, 14) aus einem zylindrischen Stück besteht, das am Ende mit einem durch eine Öffnung (15, 16) durchsetzten Kopf versehen ist, wobei das Stück mittels einer am Ende des kurzen Abschnitts (5, 6) der Arme (1, 2) ausgebildeten Bohrung frei drehbar montiert ist.

3. Biegevorrichtung nach Anspruch 1 oder 2, **dadurch gekennzeichnet, dass** die beiden Arme (1, 2) einander überlagernd angeordnet sind.

4. Biegevorrichtung nach Anspruch 3, **dadurch gekennzeichnet, dass** die an dem unteren Arm montierte Öse einen Sockel (17) mit einer der Dicke des oberen Arms entsprechenden Dicke aufweist, so dass die Mittelpunkte der Öffnungen der beiden Ösen koplanar sind.

5. Biegevorrichtung nach mindestens einem der vorhergehenden Ansprüche, **dadurch gekennzeichnet, dass** die beiden Arme (1, 2) flach sind und parallel zur Mittelebene ausgerichtet sind.

6. Biegevorrichtung nach mindestens einem der vorhergehenden Ansprüche, **dadurch gekennzeichnet, dass** der Winkel, der einerseits durch die zwischen der Drehachse einer der Ösen (13, 14) und der Drehachse (4) der Hauptrolle verlaufende Gerade und andererseits durch die zwischen der Drehachse einer Hauptrolle und dem Greifbereich des entsprechenden Arms verlaufende Achse gebildet wird, zwischen 75° und 100° beträgt.

7. Biegevorrichtung nach mindestens einem der vorhergehenden Ansprüche, **dadurch gekennzeichnet, dass** der Winkel, der einerseits durch die zwischen der Drehachse einer der Ösen und der Drehachse der Hauptrolle verlaufende Gerade und andererseits durch die zwischen der Drehachse einer Hauptrolle und dem Greifbereich des entsprechenden Arms verlaufende Achse gebildet wird, 90° beträgt.

8. Biegevorrichtung nach mindestens einem der vorhergehenden Ansprüche, **dadurch gekennzeichnet, dass** jeder Arm einen dem die Öse tragenden kurzen Abschnitt gegenüberliegenden Abschnitt aufweist, der ein vorderes Segment und ein in Bezug auf das vordere Segment nach außen hin divergierendes hinteres Segment aufweist.

9. Biegevorrichtung nach mindestens einem der vorhergehenden Ansprüche, **dadurch gekennzeichnet, dass** die Hauptrolle (4) eine Rille mit einem im Wesentlichen zu jenem der zu biegenden Stange gleichen Radius aufweist, deren Mittelpunkt mit dem Mittelpunkt der in den Ösen (13, 14) gebildeten Öffnungen koplanar ist.

10. Biegevorrichtung nach mindestens einem der vorhergehenden Ansprüche, **dadurch gekennzeichnet, dass** die Arme dazu ausgelegt sind, das Kreuzen von Zwischenbereichen der Arme zuzulassen.

11. Biegevorrichtung nach mindestens einem der vorhergehenden Ansprüche, **dadurch gekennzeichnet, dass** jeder kurze Abschnitt (5, 6) an seinem durch die Drehachse der Öse (13, 14) durchsetzten Ende eine zur Drehachse koaxiale, vertikale Verlängerung (20, 21) aufweist, deren Höhe der Dicke des kurzen Abschnitts (6, 5) des komplementären Arms entspricht, wobei der kurze Abschnitt (5, 6) ferner eine radiale Verlängerung (22, 23) umfasst, die sich parallel so zum dreieckigen Bereich zwischen der Drehachse der Öse (13, 14) und der Drehachse (3) der Hauptrolle erstreckt, dass eine die Aufnahme des dreieckigen Bereichs des komplementären kurzen Abschnitts (6, 5) zulassende Gabel derart gebildet wird, dass ein Scharnier gebildet wird.

12. Biegevorrichtung nach dem vorhergehenden Anspruch, **dadurch gekennzeichnet, dass** die vertikale Verlängerung (20), die radiale Verlängerung (22) und der kurze Abschnitt (5) monolithisch durch Gießen eines einzigen Stücks gefertigt sind.

13. Biegevorrichtung nach mindestens einem der vorhergehenden Ansprüche, **dadurch gekennzeichnet, dass** der kurze Abschnitt (5, 6) eine sich nach außen erstreckende dreieckige Form aufweist.

14. Biegevorrichtung nach mindestens einem der vorhergehenden Ansprüche, **dadurch gekennzeichnet, dass** jeder Arm einen Griff (7, 8) aufweist, wobei mindestens einer der Griffe (7, 8) Markierungen (24) aufweist, die den erreichten Krümmungsradius angeben.

15. Biegevorrichtung nach mindestens einem der vorhergehenden Ansprüche, **dadurch gekennzeichnet, dass** die Arme durch Polymergießen gefertigt sind.

## Claims

1. Cam bending machine, consisting of two arms (1, 2) hinged about a hinge axis with respect to one other, each arm comprising a short part (5, 6) that extends in front of the hinge axis, each short part (5, 6) having, at the end, means for exerting a bending force on a rod (19), the bending machine further comprising a main roller (4) that is coaxial with the hinge axis (3) of said arms (1, 2), **characterized in that** the means for exerting a bending force consist of two eyes (13, 14) rotatably mounted on the end of the short part (5, 6) of each of said arms (1, 2) about an axis of rotation that is parallel to the axis of said main roller (4), each eye (13, 14) comprising an opening (15, 16) having an axis perpendicular to the axis of rotation of the eye, the opening being arranged so as to allow the rod (19) that is to be bent to pass through.

2. Bending machine according to claim 1, **characterized in that** each eye (13, 14) consists of a cylindrical part provided, at the end, with a head through which an opening (15, 16) passes, said part being mounted so as to be freely rotatable through a bore formed at the end of the short part (5, 6) of said arms (1, 2).

3. Bending machine according to either claim 1 or claim 2, **characterized in that** the two arms (1, 2) are placed one on top of the other.

4. Bending machine according to claim 3, **characterized in that** the eye mounted on the lower arm has a base (17) of a thickness corresponding to the thickness of the upper arm, so that the centers of the openings of the two eyes are coplanar.

5. Bending machine according to at least one of the preceding claims, **characterized in that** the two arms (1, 2) are flat, and are oriented parallel to the median plane.

6. Bending machine according to at least one of the preceding claims, **characterized in that** the angle formed between the straight line passing between the axis of rotation of one of the eyes (13, 14) and the axis of rotation (4) of the main roller, and the axis passing between the axis of rotation of a main roller and the gripping zone of the corresponding arm, is between 75° and 100°.

7. Bending machine according to at least one of the preceding claims, **characterized in that** the angle formed between the straight line passing between the axis of rotation of one of the eyes and the axis of rotation of the main roller, and the axis passing between the axis of rotation of a main roller and the gripping zone of the corresponding arm, is 90°.

8. Bending machine according to at least one of the preceding claims, **characterized in that** each arm comprises a part which is opposite the short part carrying the eye and which has a front portion and a rear portion that diverge toward the outside with respect to the front portion.

9. Bending machine according to at least one of the preceding claims, **characterized in that** the main roller (4) has a groove with a radius substantially equal to that of the rod to be bent, the center of which is coplanar with the center of the openings formed in the eyes (13, 14).

10. Bending machine according to at least one of the preceding claims, **characterized in that** the arms are designed so as to allow intermediate zones of said arms to cross.

11. Bending machine according to at least one of the preceding claims, **characterized in that** each short part (5, 6) carries, at the end thereof through which the axis of rotation of the eye (13, 14) passes, a vertical extension (20, 21) that is coaxial with said axis of rotation, which extension has a height corresponding to the thickness of the short part (6, 5) of the complementary arm, said short part (5, 6) further comprising a radial extension (22, 23) that extends parallel to the triangular zone between the axis of rotation of the eye (13, 14) and the axis of rotation (3) of the main roller so as to form a fork that can receive the triangular zone of the complementary short part (6, 5) in order to form a joint.

12. Bending machine according to the preceding claim, **characterized in that** the vertical extension (20), the radial extension (22), and the short part (5) are produced so as to be integral by molding a single piece.

13. Bending machine according to at least one of the preceding claims, **characterized in that** said short part (5, 6) has a triangular shape that extends outward.

14. Bending machine according to at least one of the preceding claims, **characterized in that** each arm comprises a handle (7, 8), at least one of said handles (7, 8) having markings (24) that represent the bending radius reached.

15. Bending machine according to at least one of the preceding claims, **characterized in that** the arms are produced by polymer molding.
